# EUROPEAN PATENT APPLICATION

(19) Europäisches Patentamt

European Patent Office

Office européen des brevets

(11) Publication number: **0 508 005 A1**

(12) **EUROPEAN PATENT APPLICATION**

(21) Application number: **91303082.1**

(22) Date of filing: **08.04.91**

(51) Int. Cl.5: **C10G 47/20**, **B01J 29/02**

(43) Date of publication of application:
**14.10.92 Bulletin 92/42**

(84) Designated Contracting States:
**BE DE FR GB IT NL**

(71) Applicant: **MOBIL OIL CORPORATION**
**3225 Gallows Road**
**Fairfax, Virginia 22037-0001(US)**

(72) Inventor: **Degnan, Thomas Francis, Jr.**
**736 Paddock Path**
**Moorestown, New Jersey 08057(US)**
Inventor: **Kirker, Garry Wayne**
**18 Old Mill Road**
**Sewell, New Jersey 08080(US)**
Inventor: **Stapleton, Michael Robert**
**60 Lake Drive**
**Freehold, New Jersey 07728(US)**
Inventor: **Johnson, Ivy Dawn**
**15 Carol Joy Road**
**Medford, New Jersey 08055(US)**
Inventor: **Socha, Richard Francis**
**42 Teaberry Lane**
**Newtown, Pennsylvania 18940(US)**

(74) Representative: **Colmer, Stephen Gary et al**
**Patent Department, Mobil Court, 3 Clements Inn**
**London WC2A 2EB(GB)**

(54) **Production of hydrocarbon fractions by hydrocracking.**

(57) A process for hydrocracking 343°C+ feedstock to produce a distillate boiling-range product employs a catalyst which comprises a layered silicate, such as magadiite or kenyaite, which contains interspathic polymeric silica. The catalyst also contains at least one base metal in the form of Cr, Mo, W, Fe, Co, or Ni.

EP 0 508 005 A1

This invention is directed to the production of hydrocarbon fractions by hydrocracking.

U.S. Patent No. 4,812,222 discloses a process for producing a lubricating oil basestock by hydrocracking a hydrocarbon feedstock containing polycyclic aromatics over a pillared, layered silicate, such as pillared magadiite containing a palladium hydrogenation component. According to the present invention, it has now been found that a pillared, layered silicate is also highly selective to the production, by hydrocracking, of lower boiling, distillate range hydrocarbons.

Thus the present invention resides in a process for hydrocracking a hydrocarbon feedstock having an initial boiling point above 343°C, comprising contacting the feedstock with a catalyst having cracking and hydrogenating activity, said catalyst comprising a layered silicate containing interspathic polymeric silica and at least one metal selected from Cr, Mo, W, Fe, Co, and Ni, so as to convert in excess of 70% by volume of the feedstock to a product boiling below 343°C.

The hydrocarbon feedstock used in the process of the invention has an initial boiling point above about 343°C (650°F) and may contain polycyclic aromatics. In the latter case, the product is improved in that it contains a lesser proportion of polycyclic aromatic hydrocarbons than the feedstock which enhances the oxidation stability and viscosity index of the product.

Suitable feedstocks include heavy gas oils, residual stocks, cycle stocks, topped crudes, reduced crudes, and relatively high boiling point hydrocarbon fractions of cracking derived from coal, tars, pitches, asphalts and shale oils. These materials can be obtained by fractionation, as by vacuum distillation, of crude oils identified by their source, e.g., Pennsylvania, Mid-Continent, Gulf Coast, West Texas, Amal, Kuwait, Barco and Statfjord. Feedstocks obtained from resids should be made substantially free of asphaltenes prior to hydrocracking.

When hydrocracked, the high boiling hydrocarbon feed material employed in the present process undergoes conversion with high selectivity to lower boiling materials suitable for use as gasolines or distillates. Any polycyclic aromatic and naphthenic compounds present in the feedstock will tend to be hydrogenated and cracked to form lower molecular weight naphthenes and paraffins. If the feedstock contains waxy paraffinic components these will tend to undergo isomerization to iso-paraffins with some cracking to lower molecular weight materials. The waxy components (straight chain and slightly branched chained paraffins) are at least partially removed from the high boiling fraction so that its pour point (ASTM D-97) is decreased.

The hydrocarbon feedstock may be treated in order to reduce or substantially eliminate heteroatom content prior to the hydrocracking step. As necessary, the feedstock may be hydrotreated under mild or moderate hydroprocessing conditions to reduce sulfur, nitrogen, oxygen, and metal content in the feedstock. Generally, a hydrocarbon feedstock used in hydrocracking should have a low metals content, e.g., less than about 200 ppm, in order to avoid obstruction of the catalyst and plugging of the catalyst bed. The mild to moderate hydrotreating conditions employed include pressures of 2 to 21 MPa and $H_2$ consumptions of 20 to 280 $m^3/m^3$. Conventional hydrotreating process conditions and catalysts may be employed, e.g., those set out in U.S. Patent 4,283,272. A method for hydrotreating resids which contain naphthenes and which employs polymeric metal oxide intercalated layered oxides is set out in U.S. Patent 4,600,503.

The hydrocracking catalyst employed in the present invention comprises a layered silicate in which adjacent layers are separated by pillars containing polymeric silica. In addition, the pillars can contain polymeric oxides of one or more elements selected from B, Al, Ga, In, and Tl either separate from or incorporated into the interspathic polymeric silica pillars. Polymeric alumina is particularly useful in imparting acidic activity to the layered silicate and is typically provided as a silica-alumina pillaring material. Thus the preferred catalyst for use in the present invention contains 5 to 50 wt%, more preferably 10 to 20wt%, silica-alumina incorporated as the pillar material. The silica/alumina molar ratio ($SiO_2/Al_2O_3$) of the pillar material may vary between about 5 to 1000 or even greater.

Suitable layered silicates for producing the catalyst include the high silica alkali silicates such as magadiite, natrosilite, kenyaite, makatite, nekoite, kanemite, okenite, dehayelite, macdonaldite, and rhodesite. Unlike swellable clays, these materials lack octahedral sheets, i.e., sheets composed of atoms which are octahedrally coordinated with oxygen atoms. In contrast, these materials possess a framework composed essentially of only tetrahedral sheets, i.e., silicon is coordinated with four oxygen atoms, condensed on each other.

Although the above silicates exist naturally, their synthetic analogues can be prepared hydrothermally from an aqueous reaction mixture containing silica and caustic at relatively moderate temperatures and pressures. Moreover, the synthetic analogues can be prepared with tetracoordinate framework atoms other than Si by co-crystallization in the presence of elements such as B, Al, Ga, In, and Tl.

For example, synthetic magadiite is readily synthesized hydrothermally from a reaction mixture having the following molar composition:

$SiO_2/X_2O_3 = 10$ to infinity where X can be B, Al, Ga,

In and/or Ti or other catalytically useful metal

$\overset{+}{M}OH^-/SiO_2 = 0$ to 0.6 (preferably 0.1-0.6), where M = an alkali metal

$H_2O/SiO_2 = 8 - 500$

$R/SiO_2 = 0 - 0.4$

where R is an organic directing agent selected from benzylriethylammonium chloride, benzyltrimethylammonium chloride, dibenzyldimethylammonium chloride, N, N$^1$-dimethylpiperazine, and triethylamine. The reaction mixture is maintained at a temperature of 100 to 200°C for 1 to 150 days in order to form a product having the following composition:

% N = 0 to 3, e.g., 0 to 0.3

$SiO_2/X_2O_3 = 10$ to infinity where X may be in the tetrahedral or octahedral position

$M_2O/SiO_2 = 0$ to 0.5, e.g., 0.05 to 0.1

To produce the catalyst employed in the process of the invention, the layered silicates described above are pillared with polymeric silica so that the spacing between adjacent layers of the silicate is sufficient to allow passage of the polycyclic hydrocarbon components of the feed. The reference to polymeric silica pillars is intended to include oxides of two or more repeating units, preferably three or more repeating units, e.g., four or more or even five or more repeating units. The extent of polymerization of the interspathic polymeric silica is believed to affect the ultimate interlayer separation of the layered product; that is to say, the greater the extent of polymerization occuring, the greater the interlayer polymerization occuring, the greater the interlayer distance resulting in the pillared layered silicate. Preferably, the interlayer spacing is greater than 10 angstroms and typically is 15 to 20 angstroms.

A pillared, layered silicate suitable for use in the present hydrocracking process can be prepared according to the method set out in EP-A-205711. In this method, the interlayer spacing of the layered silicate is tailored by selection of a propping agent used to separate the layers during treatment with interspathic polymeric silica precursors, which are subsequently converted to the thermally stable polymeric silica pillars. A wide range of interlayer spacings can be achieved by this method, depending largely on the type of propping agent used as well as the layered silcate being treated.

The propping agent employed is an organic compound capable of ion exchange with or addition to interlayer cations (such hydrogen ions, hydronium ions, or alkali metal cations) associated with the layered silicate so as to separate the layers of the silicate. In particular, alkylammonium cations have been found to be useful propping agents. Thus $C_3$ and large alkylammonium cations, e.g., n-octylammonium, can be readily incorporated within the interlayer species of the layered silicates, serving to prop open the layers in such a way as to allow incorporation of the polymeric silica precursor. The extent of the interlayer spacing can be controlled by the size of the organoammonium ion employed so that use of the n-propylammonium cation can achieve an interlayer spacing of about about 2 - 5A, whereas to achieve an interlayer spacing of 10 to 20A, an n-octylammonium cation or a cation of equivalent length is required. The organic ammonium cations separating the silicate layers may also be formed in situ by reaction of the neutral amine species with hydrogen or hydronium cations of the layered silicate starting material.

After separation of the silicate layers with the propping agent, a compound capable of conversion to the polymeric oxide pillars is introduced between the silicate layers. The precursor material is preferably introduced as a cationic, or more preferably, electrically neutral, compound capable of hydrolysis to produce the polymeric pillars. The precursor material is preferably an organic compound which is a liquid under ambient conditions. In particular, hydrolyzable compounds, e.g., alkoxides, of the desired elements of the pillars are utilized as the precursors. Suitable polymeric silica precuror materials include tetraalkylsilcates, e.g., tetrapropylorthosilicate, tetramethylorthosilicate, and, most preferably, tetraethylorthosilicate. Introduction of an interspathic polymeric oxide of an element selected from B, Al, Ga, In, and Tl can be achieved by contacting a hydrolyzable compound of the desired element with the organic "propped" species before, after or simultaneously with the contacting of the layered silicate with the silicon compound. The hydrolyzable aluminum compound employed may be an aluminum alkoxide, e.g., aluminum isopropoxide.

The polymeric oxide precursor-containing product is then exposed to suitable conversion conditions, such as hydrolysis and/or calcination, to form the required pillared silicate. The hydrolysis step may be carried out by any method, for example, by interspathic water already present in the organic-"propped" layered silicate material. Because of the effect of interspathic water on hydrolysis, the extent of hydrolysis may be modified by varying the extent to which the organic-"propped" species is dried prior to addition of the polymeric oxide precursor. Calcination can be used not only to convert the polymeric oxide precursor but also to remove any organic propping agent remaining from the swelling step. After production of the polymeric oxide pillars and calcination to remove the organic propping agent, the final pillared product may contain residual exchangeable cations. Such residual cations in the layered material can be ion exchanged by known methods with other cationic species to provide or alter the catalytic activity of the pillared product. In particular, the required hydrogenation component selected from Cr, Mo, W, Fe, Co, and Ni, preferably W and Ni, can be introduced by ion-exchange. Representative ion exchange techniques are disclosed in a wide variety of patents including U.S. Patent Nos. 3,140,249; 3,140,251; and 3,140, 253. Generally, the hydrocracking catalyst of the present invention contains 0.1 to 20 wt%, preferably 0.5 to 15 wt%, of the hydrgenation component.

The pillared silicate catalyst can be composited with porous inorganic oxide matrix materials such as silica-alumina, silica-magnesia, silica-zirconia, silica-thoria, silica-beryllia, and silica-titania, as well as ternary compositions, such as silica-alumina-thoria, silica-alumina-zirconia, silica-alumina magnesia, and silica-magnesia-zirconia. The matrix may be in the form of a cogel. The relative proportions of pillared silicate component and inorganic matrix, on an anhydrous basis, may vary widely with the silicate content ranging from 1 to 99 wt%, more usually 5 to 80 wt%, of the dry composite.

The hydrocracking process can be carried out at temperatures ranging from 250 to 500°C, preferably 300 to 450°C; hydrogen pressures ranging from 2 to 21 MPa, preferably 3 to 21 MPa; liquid hourly space velocities ranging from 0.05 to 10, preferably 0.2 to 3; $H_2$ circulations ranging from 90 to 1780 $Nm^3/m^3$ (500 to 10,000 scfb), preferably 360 to 1070 $Nm^3/m^3$ (2000 to 6000 scfbl) and conversions to 650°F$^-$ (343°C-) product of greater than 75 volume% of the charge.

The catalyst possesses hydrogenation and cracking activity, the latter of which may be enhanced by the incorporation of a noble metal hydrogenation component selected from Ru, Rh, Pd, Os, Ir, and Pt, preferably palladium. These noble metals are particularly suited to applications where feeds to the hydrocracking stage are low in sulfur.

The invention will now be more particularly described with reference to the Examples and the accompanying drawings, in which:

Figure 1 compares the selectivity of a NiW/pillared kenyaite with an analogous NiW/zeolite beta catalyst for the conversion to 165-343°C (330-650°F) distillate, and

Figures 2 and 3 compare the $C_1$ to $C_4$ light gas and naphtha selectivities for NiW/pillared kenyaite and NiW/zeolite beta catalysts.

## EXAMPLE 1

Silica-alumina pillared magadiite was prepared as follows. Synthetic magadiite was used in the as-synthesized sodium form. Twelve (12) parts (dry basis) of the synthetic magadiite were reacted with 26 parts octylamine in 66 parts distilled water for 24 hrs at ambient temperature. The reaction product was treated with a flocculating agent and filtered. The resulting dry product was treated with a solution of 1 part aluminum isopropoxide in 40 parts tetraethylorthosilicate which had been prereacted at 100°C for 16 hrs and 3 days at ambient temperature; the treatment with this mixture was carried out for 48 hours at ambient temperature. The reaction product was filtered, air dried, then dried in vacuuo at about 90°C overnight. Finally, the dried product was heated at 2°C/min (5°F/min) in $N_2$ (3 v/v/min) to 540°C (1000°F) and held for 2 hours. The air flow was then slowly increased, while the $N_2$ flow was decreased, whereafter the product was held for 2 more hours in air (3 v/v/min) at 540°C (1000°F).

The silica-alumina pillared magadiite had the characteristics set forth in Table 1.

4

## TABLE 1

### Properties of Silica-Alumina Pillared Magadiite

| | |
|---|---|
| $SiO_2$, wt% | 95.1 |
| $Al_2O_3$ | 2.0 |
| N | 0.03 |
| Ash | 98.3 |
| Surface Area, $m^2/g$ | 593 |
| **Sorption** | |
| $H_2O$, g/100g | 21.5 |
| Cyclohexane | 15.9 |
| Alpha (540°C) | 5 |

The Alpha Test is described in U.S. Patent 3,354,078 and in the Journal of Catalysis, Vol. 4, p. 527 (1967); Vol. 6, p. 278 (1966); and Vol. 61, p. 395 (1980).

EXAMPLE 2

A 4 wt% of Ni/10 wt% W on silica-alumina pillared magadiite was prepared from the pillared magadiite of Example 1 by incipient wetness impregnation with ammonium metatungstate, drying at 120°C overnight, calcination in air at 1°C/min to 540°C for three hours, cooling the catalyst to room temperature, incipient wetness impregnation of the catalyst with $Ni(NO_3)_2$, drying at 120°C overnight, and calcination in air a second time at 1°C/min to 540°C for three hours.

EXAMPLE 3

The catalyst of Example 2 was charged to a reactor, presulfided, and used to convert a Statfjord distillate, with the properties shown in Table 2, at 9065 kPa (1380 psig), 0.58 LHSV. Table 3 compares the results of this run with those obtained by hydrocracking over a conventional NiW Ultrastable Y catalyst under comparable conditions. The metals loadings of both the magadiite catalyst and Ultrastable Y catalyst were equivalent.

## TABLE 2

### Properties of Statfjord Distillate

| | |
|---|---|
| Viscosity (SUS) | 150 |
| Distillation | |
| 1% | 653 (345) |
| 5% | 696 (369) |
| 50% | 784 (418) |
| 95% | 877 (469) |
| 99% | 915 (491) |
| Hydrogen, % | 13.40 |
| Nitrogen, ppm | 620 |
| Sulfur, % | 0.43 |
| Paraffins | 30.7 |
| Mononaphthenes | 16.9 |
| Polynaphthenes | 17.7 |
| Aromatics | 34.7 |
| KV 40°C, cs | 26.45 |
| KV 100°C, cs | 4.805 |
| Pour Point, °F (°C) | 95 (35) |
| Cloud Pt., °F (°C) | 112 (44) |
| Flash, COC, °C | 421 |

## TABLE 3

### Comparison Between Magadiite and USY Catalysts Of Hydrocracking of Statfjord Distillate

| Catalyst | NiW-Magadiite | NiW-USY |
|---|---|---|
| Temperature, °F (°C) | 784 (418) | 743 (395) |
| Pressure, psig (kPax10$^3$) | 1380 (9.6) | 1410 (9.8) |
| LHSV | 0.58 | 1.0 |
| Conversion, % | 81 | 78 |
| Yields, wt% | | |
| 650°F+ (343°C+) | 19 | 22 |
| 330-650°F (166-343°C) | 27 | 31 |
| $C_5$-330°F ($C_5$-166°C) | 33 | 41 |
| $C_1$-$C_4$ Gas | 21 | 6 |
| Pour Point of 650°F+ (343°C+) Fraction, °F (°C) | 45 (7) | 75 (24) |

Table 3 shows that at approximately equivalent conversions, the product from the magadiite catalyst had a significantly lower pour point than that obtained from the Ultrastable Y catalyst.

EXAMPLE 4

A silica-alumina pillared kenyaite was prepared as follows. A sample of synthetic kenyaite was prepared in a manner similar to that reported by K. Beneke and G. Lagaly in American Mineralologist, Vol. 68, p. 818 (1983). A mixture of 10.22g (87.2%) KOH and 57.4g of amorphous precipitated silica (HiSil 233) in 300g water was crystallized in a 600 ml autoclave with stirring at 150°C for 3 days. The product was filtered, washed with 4 liters water, and dried at 120°C (250°F) for one hour. The dried product had the following composition:

| | |
|---|---|
| 4.0% | K |
| 0.39% | $Al_2O_3$ |
| 68.0% | $SiO_2$ |
| 73.55% | Ash |

A sample calcined in air for one hour at 540°C (1000°F) had low surface area.

The synthetic $K^+$-form kenyaite was intercalated with cetyltrimethylammonium bromide to prop the layers apart. To do this the as-synthesized kenyaite was added directly to a IM cetyltrimethylammonium bromide solution, stirred for at least 2 hours, filtered and washed with $H_2O$. After the swollen kenyaite had been air-dried to a powder, it was impregnated lg/lg with tetraethylorthosilicate and thoroughly mixed by tumbling in a closed polypropylene jar on a roller overnight. The impregnated material (200g) was suspended in an aqueous solution of Al ($NO_3$)$_3$ (1000g, 0.5M), stirred for 18 hours, filtered, air-dried, and then calcined in air for six hours at 540°C.

The finished material had the following properties:

| Surface Area, m$^2$/g | 582 |
| Ash, 1000°C, wt% | 90.58 |
| SiO$_2$, wt% | 85.3 |
| Al$_2$O$_3$, wt% | 2.9 |
| K, ppm | 305 |

EXAMPLE 5

A ten gram sample of the material from Example 4 was charged to a polypropylene bottle. Ten milliliters of an ammonium metatungstate ((NH$_4$)$_6$W$_{12}$O$_{39}$.9H$_2$O) solution (1.63g/10cc) was added slowly, over a 5-minute period, to the powdered kenyaite sample using a pipette to impregnate the material. The bottle was agitated frequently to guarantee uniform distribution. The impregnated material was dried at room temperature overnight and then at 110°C for 24 hours. The dried material was then calcined in air by heating to 540°C at a rate of 1°C/min and holding at the final temperature for three hours.

The calcined sample was then cooled to room temperature and impregnated with a nickel nitrate solution by again charging the material to a polypropylene bottle and dropwise slowly adding 10ml of a Ni-(NO$_3$)$_2$.6H$_2$O solution (2.3g Ni (NO$_3$)$_2$.6H$_2$O/10cc). This material was dried in air at room temperature, and then in air at 110°C for 24 hours. The final step involved calcining the material in air by heating to 540°C at a rate of 1°C/min and holding at 540°C for three hours.

EXAMPLE 6

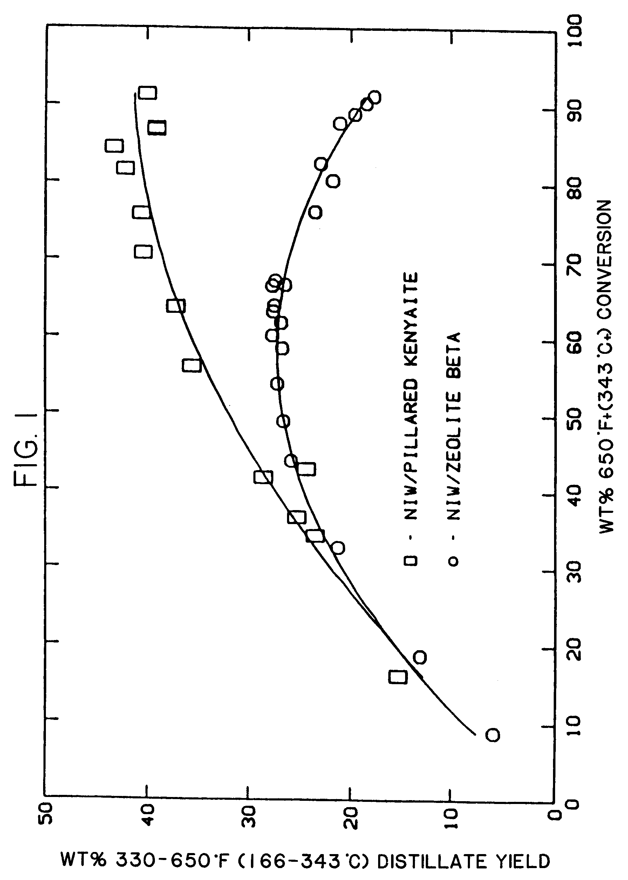

A 5.2 gram (11cc) sample of the catalyst from Example 5 was charged to a reactor and used to hydrocrack a Statfjord vacuum gas oil at 10100 kPa (1450 psig), 380 to 430°C (720 to 800°F), and 0.4 WHSV. The properties of the Statfjord vacuum gas oil are given in Table 2. Figure 1 compares the selectivity of the NiW/pillared kenyaite with an analogous NiW/zeolite beta catalyst for the conversion to 166-343°C (330-650°F) distillate. For this comparison, the NiW/zeolite beta catalyst was run under very similar conditions. These data show that the pillared kenyaite catalyst is capable of producing a higher yield of distillate than the NiW/zeolite beta catalyst. For example, at 80 wt% conversion to 343°C- (650°F-), the NiW/pillared kenyaite catalyst produced 17 wt% more distillate than the zeolite beta based hydrocracking catalyst. This result is significant, in view of the advantages of using a NiW/zeolite beta catalyst over other catalysts for maximum distillate yield as disclosed in US Patent 4,612,108.

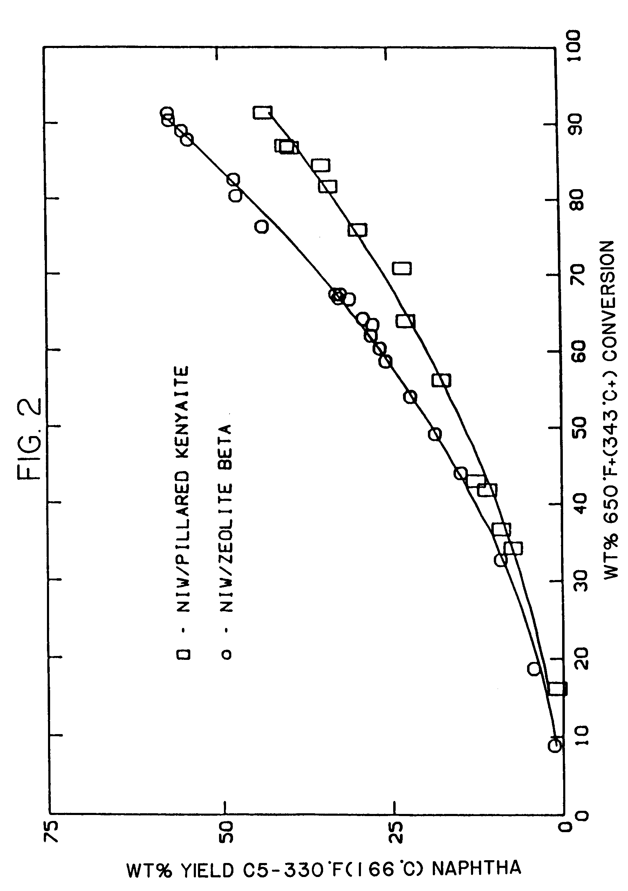

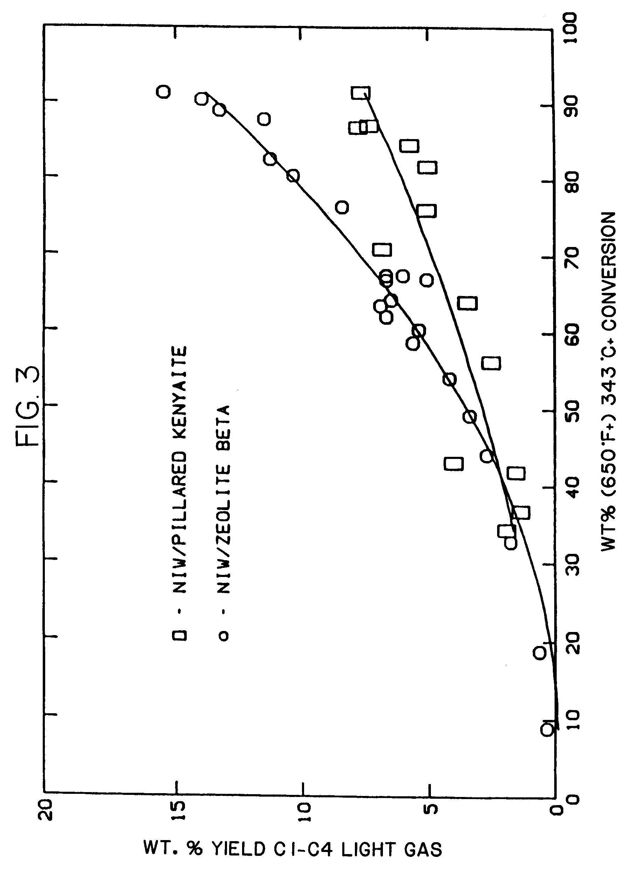

Figures 2 and 3 compare the C$_1$ to C$_4$ light gas and naphtha selectivities for the NiW/pillared kenyaite and NiW/zeolite beta catalysts. The comparatively low selectivity for light gas production is an additional highly desirable feature of the kenyaite catalyst.

Table 4 compares the product yield structures from the NiW/zeolite beta and the NiW/pillared kenyaite catalysts at about 83 wt% conversion.

EP 0 508 005 A1

## TABLE 4

|  | NiW/Zeolite Beta | NiW/Pillared Kenyaite |
|---|---|---|
| Temperature, °F (°C) | 721 (383) | 766 (408) |
| LHSV, hr$^{-1}$ | 0.52 | 0.22 |
| WHSV, hr$^{-1}$ | 0.75 | 0.39 |
| H$_2$ flow, scf/BBL (Nm$^3$/m$^3$) | 4600 (820) | 6100 (1086) |
| Conversion, wt% | 83 | 82 |
| Yields, wt% |  |  |
| 330-650°F (166-343°C) dist. | 27 | 42 |
| C$_5$-330°F (166°C)naph. | 48 | 34 |
| C$_1$-C$_4$ Gas | 11 | 5 |

**Claims**

1. A process for hydrocracking a hydrocarbon feedstock having an initial boiling point above 343°C, comprising contacting the feedstock with a catalyst having cracking and hydrogenating activity, said catalyst comprising a layered silicate containing interspathic polymeric silica and at least one metal selected from Cr, Mo, W, Fe, Co, and Ni, so as to convert in excess of 70% by volume of the feedstock to a product boiling below 343°C.

2. A process according to claim 1, wherein said layered silicate further contains an interspathic polymeric oxide of an element selected from Al, B, Cr, Ga, In, Mo, Nb, Ni, Ti, Tl, W, and Zr.

3. A process according to claim 1, wherein said layered silicate further contains an interspathic polymeric oxide of Al.

4. A process according to any preceding claim, wherein said hydrocracking occurs at a temperature of 250 to 500°C, a hydrogen pressure of 2 to 21 MPa, a liquid hourly space velocity of 0.05 to 10 and a H$_2$ circulation rate of 90 to 1780 Nm$^3$/m$^3$ (50 to 10,000 scfb).

5. The process according to any preceding claim, wherein said hydrocarbon feedstock is hydrotreated prior to said hydrocracking.

6. A process according to any preceding claim, wherein said layered silicate comprising interspathic polymeric silica has an interlayer separation greater than 10 angstroms.

7. A process according to any preceding claim, wherein said layered silicate is selected from magadiite, natrosilite, kenyaite, makatite, nekoite, kanemite, okenite, dehayelite, macdonaldite and rhodesite.

9

## FIG. 1

WT% 330-650°F (166-343°C) DISTILLATE YIELD

WT% 650°F+(343°C+) CONVERSION

□ - NIW/PILLARED KENYAITE

○ - NIW/ZEOLITE BETA

EP 0 508 005 A1

FIG. 2

WT% YIELD C5-330°F(166°C) NAPHTHA

WT% 650°F+(343°C+) CONVERSION

□ - NIW/PILLARED KENYAITE

o - NIW/ZEOLITE BETA

EP 0 508 005 A1

FIG. 3

□ - NIW/PILLARED KENYAITE

o - NIW/ZEOLITE BETA

WT. % YIELD C1-C4 LIGHT GAS

WT% (650°F+) 343°C+ CONVERSION

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,Y | US-A-4 812 222 (MOBIL OIL CORPORATION)<br>* column 4, line 34 - line 39; claims 1-4,7-10,13-15 *<br>--- | 1-7 | C10G47/20<br>B01J29/02 |
| Y | EP-A-0 302 790 (COMPAGNIE DE RAFFINAGE ET DE DISTRIBUTION TOTAL FRANCE)<br>* page 3, line 17 - line 20; claims 1-4 *<br>* examples 1,2 *<br>--- | 1-7 | |
| A | WO-A-8 806 614 (UNION CARBIDE)<br>* claims 1,15,17-18,27,28,50 *<br>--- | 1-7 | |
| A | US-A-4 510 257 (SHELL OIL)<br>* column 8, line 25 - line 55; claims 1,13,14 *<br>--- | 1-7 | |
| A | US-A-4 367 163 (RESEARCH CORPORATION)<br>* claims 1-28 *<br><br>----- | 1-7 | |
| | | | TECHNICAL FIELDS SEARCHED (Int. Cl.5 )<br><br>B01J<br>C10G |

The present search report has been drawn up for all claims

| Place of search | Date of completion of the search | Examiner |
|---|---|---|
| THE HAGUE | 05 DECEMBER 1991 | MICHIELS P. |

EPO FORM 1503 03.82 (P0401)